# EUROPÄISCHE PATENTSCHRIFT

(11) **EP 3 655 088 B1**
(45) Veröffentlichungstag und Bekanntmachung des Hinweises auf die Patenterteilung: **13.03.2024**
(21) Anmeldenummer: 18743468.3
(22) Anmeldetag: 18.07.2018
(51) Int. Cl.: A61M 39/28, A61M 5/142

(54) **VORRICHTUNG UND VERFAHREN ZUM ÖFFNEN UND SCHLIESSEN EINER INFUSIONSSCHLAUCHKLEMME**
APPARATUS AND METHOD FOR OPENING AND CLOSING AN INFUSION TUBE CLAMP
DISPOSITIF ET PROCÉDÉ POUR L'OUVERTURE ET LA FERMETURE D'UNE PINCE DE TUYAU DE PERFUSION

(30) Priorität: 18.07.2017 DE 102017116106
(43) Veröffentlichungstag der Anmeldung: 27.05.2020
(73) Patentinhaber: B. Braun Melsungen AG, 34212 Melsungen (DE)
(72) Erfinder: GERLACH, Hans-Josef, 34431 Marsberg (DE); STEGER, Jürgen, 34327 Körle (DE)
(74) Vertreter: Winter, Brandl - Partnerschaft mbB
(86) Internationale Anmeldenummer: PCT/EP2018/069544
(87) Internationale Veröffentlichungsnummer: WO 2019/016283

(56) Entgegenhaltungen:
- WO-A1-2011/121923
- JP-A- 2004 073 822
- US-A1- 2009 306 592

## Beschreibung

Die Erfindung betrifft eine Vorrichtung und ein Verfahren zum Öffnen und Schließen einer Infusionsschlauchklemme, wobei beim Verschließen und Öffnen einer Klappe die Infusionsschlauchklemme geschlossen wird und in der Endschließposition der Klappe geöffnet werden kann. Aufweisend eine Schieberkulisse mit einem Verriegelungselement, das eine Schieberkulissenrampe hat und einem Schließblech, das ein Schaltnocken aufweist und mit einem schwenkbaren Klemmschenkel einer Infusionsschlauchklemme verbunden ist.

### Hintergrund der Erfindung

In der Medizintechnik sind Infusionsschlauchpumpen bekannt, bei denen das zu fördernde Medium durch äußere mechanische Verformung eines Schlauches mittels eines Rotorkopfes durch diesen hindurchgedrückt wird (Quetschpumpenprinzip). Bei derartigen Pumpen werden standardmäßig Sicherheits- oder Schlauchklemmen an dem Schlauch eingesetzt, um einen sogenannten "Free-Flow" zu verhindern, kurz FFC (free-flow clamp). Unter der Bezeichnung "Free-Flow" ist dabei der Gravitationsfluss zu verstehen, der eventuell in einer gefährlichen Überinfusion resultieren kann. Die Klemmen befinden sich standardmäßig direkt am Infusionsschlauch, um einen Durchfluss eines Infusionsfluides durch den Infusionsschlauch zu unterbinden oder zu ermöglichen. Zum Einlegen eines Infusionsschlauches in eine Infusionspumpe wird meist eine an einem Pumpengehäuse anscharnierte Pumpenklappe geöffnet und bei stationären Sicherheitsklemmen in der Infusionspumpe der Schlauch in diese eingelegt.

Im Allgemeinen werden Sicherheitsklemmen meist indirekt durch eine manuelle Betätigung der Pumpenklappe der Infusionspumpe zwangsmäßig geöffnet oder geschlossen, um quasi automatisch ein ungewolltes Durchfließen des Fluids durch den Infusionsschlauch zu unterbinden. Hierbei kann es auch vorkommen, dass die Sicherheitsklemme direkt manuell geschlossen werden muss, bevor die Pumpenklappe ordnungsgemäß geschlossen werden kann. Oftmals verhält es sich aber so, dass die schlauchseitige Sicherheitsklemme zuerst direkt manuell geschlossen wird und dann aber mit dem Schließen der Pumpenklappe wieder indirekt (automatisch) geöffnet wird. Eine nach dem Schließen der Pumpenklappe geschlossene Schlauchklemme kann mittels eines Aktuators der Infusionspumpe für den Pumpenbetrieb automatisch wieder direkt geöffnet werden. Neben Infusionspumpen mit einer derartigen Öffnungsfunktion existieren Aktuatoren für eine entsprechende Schließfunktion zum Schließen der schlauchseitigen Sicherheitsklemme.

### Stand der Technik

Die EP 0 813 430 B1 offenbart einen Flusstopmechanismus, der automatisch eine flexible Röhre schließt, wenn sich eine Kassette nicht in einer Pumpe befindet. Wenn sich keine Kassette in der Pumpe befindet, kann der Schlauch durch manuelles Betätigen eines Pins geöffnet werden.

Die EP 2 583 716 A1 offenbart eine Infusionsschlauchklemme sowie ein Verfahren zum Verwenden der Infusionsschlauchklemme. Die Schlauchklemme umfasst einen Grundkörper, mit einem Infusionspumpenaufnahmebereich, sowie einem ersten Klemmschenkel und einem zweiten Klemmschenkel zum Quetschen des aufgenommenen Infusionsschlauches. Die Klemmschenkel sind an einem Ende schwenkbar miteinander verbunden. Die Infusionsschlauchklemme weist eine Schnappeinrichtung zum sicheren Positionieren der beiden Klemmschenkel in mehr als einer Lage auf.

Die Druckschriften JP 2004 073 822 A und WO 2011 121 923 A1 zeigen eine Transfusionspumpe, bzw. eine Infusionspumpe, mit einer Schlauchklemme, die beim Einsetzen in das Gehäuse der Pumpe einen Schlauch abklemmt, wobei das Schließen einer Gehäusetür der Pumpe die Klemme - und damit den Transfusionsschlauch - öffnet.

Die Druckschrift US 2009 030 592 A1 zeigt eine Infusionspumpe mit einem Infusionsschlauch, dessen Strömungsquerschnitt sowohl mittels einer Ventilanordnung als auch einer Schiebeklemme in Abhängigkeit einer Schließstellung eines Türverriegelungsmechanismus auf- und zusteuerbar ist.

Wenn gemäß dem bekannten Stand der Technik die Schlauch- oder Sicherheitsklemme als Einmalartikel ausgeführt ist, kann es - bedingt durch Toleranzen in der Herstellung der Schlauchklemme - insbesondere bei einer indirekten Betätigungsbauart z. B. über die Betätigung der Pumpengehäuseklappe ggf. zu einem nicht vollständigen Schließen (oder Öffnen) der Schlauchklemme und damit zum "Free-Flow" kommen. Auch ist es so, dass Sicherheitsklemmen der Einwegbauart mit Toleranzen in das Pumpengehäuse eingesetzt werden müssen, sodass das zumeist mechanische Zusammenspiel zwischen Klemmenmechanismus und Betätigungsmechanismus beispielsweise der an das Pumpengehäuse anscharnierten Klappe nicht optimal funktioniert.

### Kurzbeschreibung der Erfindung

Es ist daher die Aufgabe der vorliegenden Erfindung, eine Vorrichtung zum Öffnen und/oder Schließen einer Infusionsschlauchklemme bereitzustellen, die ein sicheres Öffnen und Schließen ermöglicht und geringe Toleranzen in der Herstellung hat bzw. unempfindlich gegenüber Toleranzen auf Seiten der Schlauch-/Sicherheitsklemme (konzipiert als Einwegartikel) ist. Ein weiteres Ziel der Erfindung ist die einfache Handhabung und Verringerung der Möglichkeit menschlichen Versagens.

Diese Aufgabe wird durch die Merkmale der Vorrichtung nach Anspruch 1 und demVerfahren nach Anspruch 7 gelöst. Weitere vorteilhafte Ausführungsformen sind in den Unteransprüchen beansprucht.

Das Grundkonzept der vorliegenden Erfindung sieht demnach eine(n) Schließvorrichtung (Schließmechanismus) für eine an ein Pumpengehäuse anscharnierte Klappe vor, der gleichzeitig auch das (indirekte) Betätigen (Schließen und Öffnen) zumindest einer im Pumpengehäuse fixierten/fixierbaren Sicherheits-/Schlauchklemme (vorzugsweise der Einwegartikelbauart) bewirkt. Erfindungsgemäß ist in dem Betätigungs-/Klemmkraftzug zwischen dem Schließmechanismus und der Sicherheitsklemme ein elastisches Bauteil mit bestimmter Federsteifigkeit angeordnet, das Toleranzen im Betätigungskraftzug beispielsweise infolge Herstellungs- und/oder Montagetoleranzen insbesondere auf Seiten der Sicherheitsklemme ausgleicht. Die Federsteifigkeit ist dabei so gewählt, dass bei Betätigen der Schließvorrichtung eine Klemmkraft auf die Schlauch-/Sicherheitsklemme aufgebracht wird, die maximal zu einem vollständigen Abdrücken eines darin eingelegten Schlauchs führt.

Grundsätzlich hat die in der Erfindung eingesetzte Sicherheitsklemme in Übereinstimmung mit dem Stand der Technik zwei vorzugsweise schwenkend relativ zueinander bewegbare Klemmelemente oder Klemmbacken, die in (geschlossener) Klemmposition über eine an einer Federzunge ausgebildeten/angeordneten Rastnase miteinander verrasten. Zum Öffnen der Sicherheitsklemme ist ebenfalls in Übereinstimmung mit dem Stand der Technik ein Aktuator (z. B. eine Kolben-ZylinderAnordnung) vorgesehen, der gegen die Rastnase drückt und diese aus der Rastposition bewegt.

Der erfindungsgemäße Schließmechanismus hat nunmehr einen an der Klappe gelagerten oder lagerbaren Schieber oder Riegel mit einer zumindest einen offenen Schlitz bildenden Kulisse, die bei einem Axialverschieben des Riegels in Richtung Schließposition zumindest einen am Pumpengehäuse angeordneten/ausgebildeten Zapfen oder Bolzen (Riegelschloss) hinterschneidet und somit der Axialverschiebung des Riegels in Richtung Schließposition die Klappe gegen das Pumpengehäuse zunehmend anzieht.

Am Riegel ist ferner ein Betätigungselement, vorzugsweise eine Andrückplatte/Schließblech mit innerer Elastizität oder einem internen Federmechanismus montiert, vorzugsweise schwenkbar sowie relativ axialverschiebbar angelenkt, das sich bei einer Axialverschiebung des Riegels in Richtung Schließposition (über eine daran ausgebildete Schaltnocke) gegen einen rampenförmigen Anschlag (Verriegelungselement mit daran ausgebildeter Schieberkulissenrampe) am Riegel abstützt und so bei einer Axialverschiebung des Riegels relativ zum Betätigungselement auf wenigstens eine Klemmbacke der Sicherheitsklemme eine (zunehmende federelastische) Drückkraft ausübt, um diese in Richtung Klemmposition zu bewegen, bis die Rastnase eingerastet ist.

Spätestens mit Erreichen der maximalen Schließposition des Riegels überfährt der riegelseitige Anschlag das Betätigungselement vollständig und gibt somit das Betätigungselement, vorzugsweise die Andrückplatte frei. Die Sicherheitsklemme kann nun bei vollständig geschlossener und verriegelter Klappe mittels des Aktuators problemlos geöffnet werden, um einen Fluidstrom freizugeben.

Zum Öffnen der Klappe wird der Riegel aus der Schließposition zurückgezogen, wobei der riegelseitige Anschlag sich erneut gegen das Betätigungselement anlegt, wodurch die eine Klemmbacke der Sicherheitsklemme in die Klemmposition (federelastisch) gedrückt wird, in welcher die Rastnase erneut einrastet. Die Klappe kann nun vollständig entriegelt und geöffnet werden, ohne, dass die eingerastete Sicherheitsklemme öffnet.

In anderen Worten ausgedrückt weist die Vorrichtung zum Öffnen und Schließen einer (Infusions-)schlauchklemme in einer bevorzugten Ausführungsform eine Schieberkulisse (Riegel) auf, die axial an einer Klappe eines Pumpengehäuses verschiebbar angeordnet/anordenbar ist und wenigstens eine Handhabe zum Schließen der Schlauchklemme hat. Des Weiteren weist die Vorrichtung die (Infusions-)Schlauchklemme mit einem ersten Klemmschenkel (Klemmbacke) und zweiten Klemmschenkel (Klemmbacke) auf, die an einem Ende schwenkbar miteinander verbunden sind, und ein Betätigungselement/Schließblech, das an dem ersten Klemmschenkel angeordnet ist. Die Schieberkulisse weist des Weiteren eine Schieberkulissenrampe (Anschlag) auf, an der eine Schaltnocke des Schließblechs bei einer Axialverschiebung der Schieberkulisse relativ zum Schließblech abgleitet und schließlich in eine spannungsfreie Endschließposition gleitbar ist.

In einer bevorzugten Ausführungsform ist es vorgesehen, dass die Schieberkulisse/Schieber/Riegel über ein Hebelgelenk/Kniehebel axial bewegbar ist, das wiederum mit einem Griff/Handgriff verbunden ist. Die Rotationsbewegung des Griffs wird somit in eine Axialbewegung der Schieberkulisse umgewandelt. Die Schieberkulisse ist axial an Ihrer Längsachse verschiebbar, wobei die Schieberkulisse bevorzugt an einem oberen Rand der Klappe/Tür oder dergleichen aufliegt und die Axialrichtung somit parallel zur Klappenfront/Klappendeckelvorderseite ist.

In einer weiteren Ausführungsform weist die Infusionsklemme bei einemgeschlossenem Zustand verschiedene Stellungen auf, insbesondere eine spannungsbeaufschlagende Stellung und eine spannungsfreie Stellung. Das Betätigungselement/Schließblech ist in der spannungsbeaufschlagenden Stellung durch das Verriegelungselement/Anschlag in druckbeaufschlagenden Eingriff und in der spannungsfreien Stellung mit dem Verriegelungselement/Anschlag in drucklosem Eingriff. In anderen Worten wird die Klappe geschlossen und das Verriegelungselement/Anschlag ist gegen das Schließblech/Betätigungselement drückbar/in Eingriff, weshalb sich dieses in einer spannungsbeaufschlagenden Stellung befindet, wobei der spannungslose Zustand auch als spannungsärmer oder Kontakt ohne Druck verstanden werden kann. Die spannungslose Stellung wird in der Schließendposition erreicht.

In einer weiteren Ausführungsform ist die Infusionsschlauchklemme bei geschlossener Klappe in der spannungsbeaufschlagenden Stellung schließend und bei der spannungsfreien Stellung öffenbar. In anderen Worten ist die Infusionsschlauchklemme durch die Schieberkulisse schließbar und somit in einer spannungsbeaufschlagenden Stellung und durch die Verschiebbarkeit der Schieberkulisse in die Endschließposition, bei der die Schieberkulisse außer Eingriff mit dem Schließblech ist, öffenbar, kann also geöffnet werden, ist also in einer spannungsfreien Stellung.

In einer weiteren Ausführungsform ist das Verriegelungselement/Anschlag an der Schieberkulisse in Form eines Winkelelements ausgebildet, vorzugsweise mit einem Winkel von im Wesentlichen 90°. In anderen Worten ist die Schieberkulisse flach und annähernd bandförmig (Blechbauteil). Der Querschnitt im Bereich des Verriegelungselements, das nicht durchgehend in Längsrichtung der Schieberkulisse ist, hat eine Winkelform. Das Verriegelungselement/Anschlag ist somit einstückig mit der Schieberkulisse in L-Form ausgebildet. Der Anschlag kann senkrecht also im 90°-Winkel sein, er kann aber auch jede andere Form oder einen anderen Winkel zum Betätigen des Schließblechs aufweisen.

In einer weiteren Ausführungsform ist das Schließblech ein, vorzugsweise einstückiges, Federblech (Blattfeder). In anderen Worten kann das Schließblech eine innere Federwirkung aufweisen. Es ist aber auch möglich, das Schließblech aus zwei Elementen/Platten zu bilden, die relativ bewegbar miteinander verbunden sind (z. B. per Scharnier), wobei zwischen den beiden Elementen/Platten ein separates Federelement angeordnet ist, sodass das Schließblech eine interne Federwirkung erhält.

In einer weiteren Ausführungsform weist die Vorrichtung einen elektrisch oder manuell betätigbaren Aktuator auf. Wenn sich die Infusionsschlauchklemme in einem geschlossenen Zustand befindet und die Rat-/Schnappnase an der Klemme diese verriegelt, ist der im Pumpengehäuse vorhandener Aktuator dafür vorgesehen und angepasst, diese Schnappnase zu betätigen und die Infusionsschlauchklemme zu öffnen, wobei das Öffnen der Infusionsschlauchklemme/das Aufschwenken der Klemmschenkel nur möglich ist, wenn sich das Schließblech in der Endschließposition in einem axialen Bereich der Schieberkulisse befindet, der kein Verriegelungselement/Anschlag aufweist.

In einer weiteren Ausführungsform ist die Schieberkulissenrampe in ständigem, gleitendem Kontakt mit der Schaltnocke. In anderen Worten weist die Schaltnocke einen abgeschrägten, gespanten Bereich auf, der auch in der Endschließposition noch in Kontakt mit dem Verriegelungselement/Anschlag ist. In dieser Position ist die Schaltnocke des Schließblechs noch in Kontakt mit der Schieberkulissenrampe des Verriegelungselements/Anschlags der Schieberkulisse, wird durch diese jedoch nicht mehr druckbeaufschlagt. Dieser Kontakt dient lediglich dazu, dass die Schaltnocke beim Öffnen der Klappe bzw. bei Entriegeln der Klappe wieder in/auf das Verriegelungselement/Anschlag gleiten kann. Alternativ dazu kann die Infusionsschlauchklemme auch einen Klappwinkelbegrenzer aufweisen, der den ersten Klappenschenkel daran hindert, komplett aufzuklappen, weshalb hierbei auch kein Kontakt mehr zwischen Schieberkulisse und Schließblech nötig ist.

In einer bevorzugten Ausführungsform wird ein Verfahren zum Öffnen und Schließen einer Infusionsschlauchklemme aufgezeigt, bei dem beim Verschließen und Öffnen einer Klappe des Pumpengehäuses die Infusionsschlauchklemme geschlossen wird und in der Endschließposition der Klappe geöffnet werden kann. Die Klappe wird geschlossen und ein Verriegelungselement/Anschlag einer axial verschiebbaren Schieberkulisse gegen einen Schaltnocken eines (federelastischen) Schließblechs/Betätigungselements gedrückt, wodurch eine Infusionsschlauchklemme geschlossen wird. Die Schaltnocke gleitet an einer Schieberkulissenrampe zum Erreichen der Endschließposition ab, worauf das Schließblech nicht mehr mit Kraft beaufschlagt wird. Ein Aktuator kann die Infusionsschlauchklemme in der Endschließposition der Schieberkulisse öffnen. Die Infusionsschlauchklemme wird beim Öffnen der Klappe durch die reverse axiale Verschiebung der Schieberkulisse (automatisch/zwangsweise) geschlossen.

In anderen Worten ausgedrückt wird die Klappe geschlossen, die Schieberkulisse drückt dabei gegen das Schließblech und schließt somit auch die Infusionsschlauchklemme, während sich die Schieberkulisse in Richtung Endschließposition bewegt. Dabei drückt das Verriegelungselement der Schieberkulisse gegen die Schaltnocke des Schließblechs, das an dem ersten Klemmschenkel der Infusionsklemme angeordnet ist/anliegt. Der erste Klammschenkel klappt somit zusammen mit dem Schließblech gegen den zweiten Klemmschenkel und schließt die Klemme bzw. schließt eine Öffnung in einem Infusionsschlauch durch Abquetschen. Die Schaltkulisse bewegt sich weiter in eine Axialrichtung und somit gleitet die Schaltnocke an der Schieberkulissenrampe ab und das Schließblech wird entlastet. Da das Schließblech nun die Infusionsschlauchklemme nicht mehr zusammen drückt, kann diese nun durch den Aktuator geöffnet werden. Unter Öffnen ist dabei zu verstehen, dass der Infusionsschlauch zwischen den Klemmschenkeln nicht mehr vollständig eingequetscht ist und eine Durchgangsöffnung für Infusionsfluid aufweist. Die Schaltnocke des Schließblechs gleitet vor dem Erreichen der Endschließposition der Schieberkulisse an der Schieberkulissenrampe an der Schieberkulisse ab, wobei dies realisiert wird durch den abgeschrägten Bereich der Rampe und der Schaltnocke. In der Endschließposition der Schieberkulisse kann die Schaltnocke noch in (losem) Kontakt mit der Rampe sein, aber auch freistehend.

### Kurzbeschreibung der Figuren

In einer bevorzugten Ausführungsform erreicht die Schieberkulisse durch Verschieben in einer Axialrichtung die Endschließposition. Die Schieberkulisse ist axial verschiebbar auf/an einer Randfläche der Klappe gelagert.

Die Erfindung wird unter Bezugnahme auf die Zeichnungen, in welcher eine Ausführungsform beispielhaft dargestellt ist, näher beschrieben.
Fig. 1 zeigt eine perspektivische Ansicht der Komponenten der Vorrichtung ohne Gehäuse
Fig. 2 zeigt eine Schnittansicht durch die Vorrichtung in dem Gehäuse in einer ersten Schließposition (offene Klappenstellung).
Fig. 3 zeigt eine Draufsicht auf die Komponenten in einer ersten Schließposition (offene Klappenstellung).
Fig. 4 zeigt eine Schnittansicht durch die Vorrichtung in dem Gehäuse in einer zweiten Schließposition (halboffene Klappenstellung).
Fig. 5 zeigt eine Draufsicht auf die Komponenten in einer zweiten Schließposition (halboffene Klappenstellung).
Fig. 6 zeigt eine Schnittansicht durch die Vorrichtung in dem Gehäuse in einer dritten Schließposition (geschlossene Klappenstellung).
Fig. 7 zeigt eine Draufsicht auf die Komponenten in einer dritten Schließposition (geschlossene Klappenstellung).
Fig. 8 zeigt eine Schnittansicht durch die Vorrichtung in dem Gehäuse in einer vierten Schließposition (end Klappenstellung).
Fig. 9 zeigt eine Draufsicht auf die Komponenten in einer vierten Schließposition (end Klappenstellung).

### Figurenbeschreibung

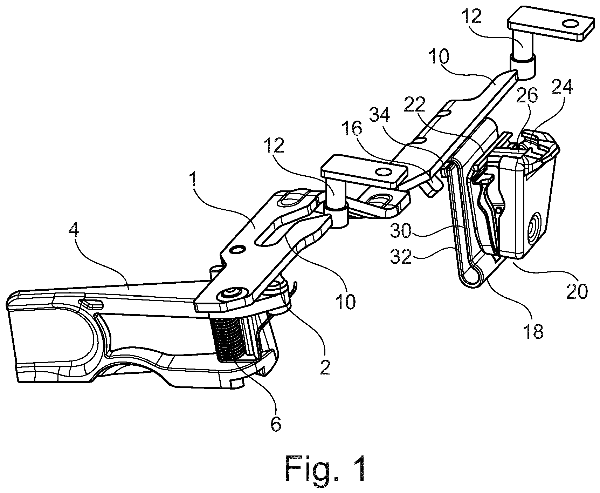

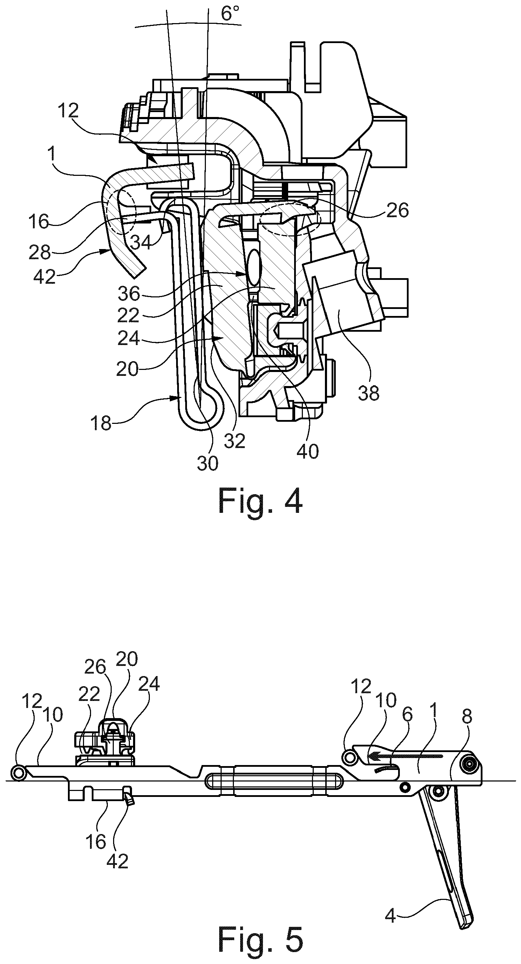

In Figur 1 ist eine perspektivische Ansicht der Komponenten einer erfindungsgemäßen Vorrichtung bzw. eines erfindungsgemäßen Mechanismus zum Öffnen und Schließen einer (Infusions-)Schlauchklemme ohne vollständiges Gehäuse einer nicht weiter dargestellten (Infusions)-Pumpe vorzugsweise der Quetschpumpenbauart gezeigt. Demzufolge hat die Vorrichtung eine(n) an einer Klappe (siehe Fig. 4) eines Infusionspumpengehäuses (siehe Fig. 4) gelagerten bzw. lagerbaren Schieberkulisse oder Schieber/Riegel 1, die über ein Hebelgelenk 2 mit einem Griff/Klappengriff 4 verbunden ist. Der Griff 4 ist im Bereich des Hebelgelenks 2 mit der nicht weiter dargestellten Klappe des Infusionspumpengehäuses schwenkbar gekoppelt bzw. koppelbar und ist mittels einer Feder/Grifffeder 6 in einer ersten Schwenkrichtung (Schließrichtung) vorgespannt. Eine Dreh-/Schwenkbewegung des Griffs 4 wird somit durch das Hebelgelenk 2 in eine Axialbewegung der Schieberkulisse 1 längs der Klappe umgewandelt. Die Schieberkulisse 1 kann somit entlang ihrer Längsachse 8 durch Verschwenken des Griffs 4 mit einem entsprechend der Konstruktion des Kniehebelgelenks 2 bestimmten Übersetzungsverhältnisses verschoben werden. Durch die Verschiebung der Schieberkulisse 1 kommen in der Schieberkulisse 1 ausgebildeten Führungsnuten 10 in Eingriff mit Zapfen/Bolzen vorzugsweise mit Rollen 12, die an dem Gehäuse/Infusionspumpengehäuse des Infusionspumpengehäuses angebracht sind. Durch diesen Eingriff ist die Schieberkulisse 1 und somit die nicht weiter gezeigte Klappe an dem die Schieberkulisse 1 gelagert ist, zum ebenfalls nicht detailliert gezeigten Gehäuse ziehbar. Die Schieberkulisse 1 weist einen Bereich auf, an dem ein Anschlag/Verriegelungselement 16 ausgebildet ist. Das Verriegelungselement 16 ragt nach unten, also entgegen dem Klappenrand und ist vorzugsweise in der Form von einem gebogenen Vorsprung an der Schieberkulisse 1 ausgebildet. In dem Bereich in dem das (zumindest eine) Verriegelungselement 16 ausgebildet ist, ist auf der korrespondierenden Seite des Infusionspumpengehäuses, also bei geschlossener Klappe 14 direkt gegenüberliegend ein Betätigungselement/Schließblech 18 angeordnet, das an einer Infusionsschlauchklemme 20 anliegt. Die Schieberkulisse 1 bewegt sich somit in seiner axialen Längsrichtung, während die Infusionsschlauchklemme 20 und die Zapfen 12 ortsfest/stationär an dem Gehäuse angebracht sind. Das Schließblech 18 ist, wie die Fig 1 zeigt, bevorzugt an der Schieberkulisse 1 anscharniert, derart, dass die Schieberkulisse 1 relativ zum Schließblech 18 axialverschiebbar ist. Alternativ kann das Schließblech 18 aber auch am Pumpengehäuse oder an der Klappe angelenkt/montiert sein.

Die Infusionsschlauchklemme 20 weist einen ersten Klemmschenkel 22 und einen zweiten Klemmschenkel 24 auf. Der zweite Schenkel 24 ist an dem Gehäuse der Infusionspumpe angebracht und an einem unteren Ende mit dem ersten Klemmschenkel 22 klappbar/schwenkbar verbunden. Da das Schließblech 18 an dem ersten Klemmschenkel 22 lediglich anliegt, ist auch das Schließblech 18 relativ zu dem zweiten Klemmschenkel 24 und somit zu dem Gehäuse der Infusionspumpe schwenkbar. Des Weiteren weist die Infusionsschlauchklemme 20 eine Schnapp- oder Rastnase 26 an ihrem oberen Ende auf, so dass der erste Klemmschenkel 22 mit dem zweiten Klemmschenkel 24 in einer Klemmenschließposition verrasten kann.

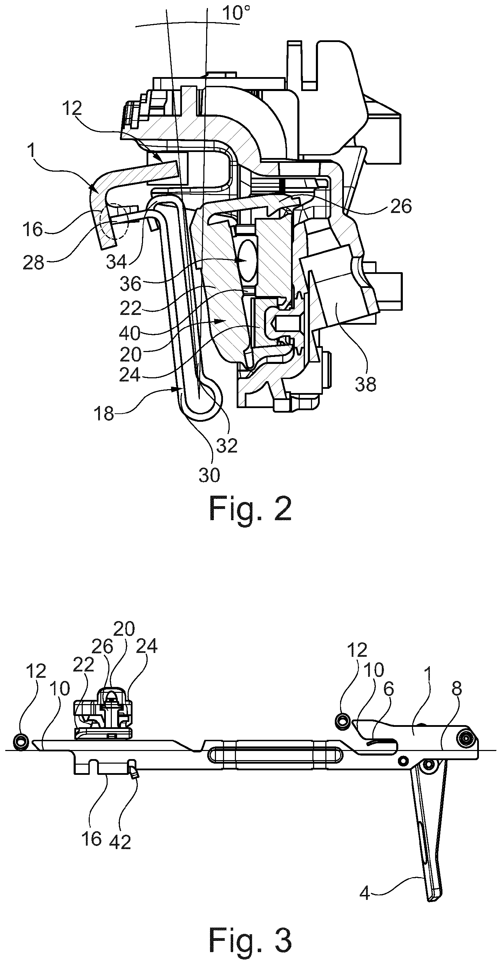

Fig. 2 zeigt eine Schnittansicht durch die Vorrichtung in dem Gehäuse in einer ersten Schließposition (offene Klappenstellung), genauer gesagt in einer Position, in der der erste Klemmschenkel 22 einen Winkel von vorzugsweise 10° relativ zu dem zweiten Klemmschenkel aufweist. Das bedeutet, die Klappe ist im Wesentlichen geschlossen, aber der Griff 4 wurde noch nicht betätigt. In dieser Ansicht ist eine Schaltnocke 28 sichtbar, die sich von dem Schließblech 18 in Richtung hin zu der geschlossenen Klappe erstreckt und mit dem Anschlag/Verriegelungselement 16 in Anlage ist. Das Schließblech 18 weist im Übrigen einen ersten Schließfederblechschenkel 30 und einen zweiten Schließfederblechschenkel 32 auf, die U- oder V-förmig miteinander vorzugsweise stoffeinstückig verbunden sind. Der zweite Schließblechschenkel 32 ist an dem ersten Klemmschenkel 22 befestigt und an einem unteren Ende mit dem ersten Schließblechschenkel 30 derart verbunden, dass er nach dem Federprinzip schwenkbar ist. An dem ersten Schließblechschenkel 30 ist die Schaltnocke 28 einstückig in einem vorbestimmten Winkel, vorzugweise 90°, abstehend angeordnet. An dem zweiten Schließblechschenkel 32 ist ein Umgriff oder Öse 34 ausgebildet, der vorzugsweise halbkreisförmig ausgebildet ist, und in den ersten Schließblechschenkel 30 eingreift, um diesen Öffnungswinkel zu begrenzen. Das bedeutet, dass der Umgriff 34 des zweiten Schließblechschenkels 32 den ersten Schließblechschenkel 30 in Eingriff nimmt und diesen unter Spannung hält und ein Aufklappen des Schließblechs 18 verhindert.

Außerdem kann der Umgriff 34 als Scharnier zur Schwenkkopplung mit der Schieberkulisse 1 ausgeformt sein.

In der ersten Schließposition ist des Weiteren zu sehen, wie der Infusionsschlauch 36 in einem offenen/ungequetschten Zustand zwischen den Klemmschenkeln 22 und 24 liegt. Ferner ist in dieser Ansicht auch ein Aktuator 38 zum Öffnen der Schnappnase 26 ersichtlich. Der Aktuator 38 kann von unten an das über die Infusionsschlauchklemme 20 überstehende Ende der Schnappnase 26 drücken und diese dadurch entrasten und somit auch die Infusionsschlauchklemme 20 öffnen, wie in einem späteren Schritt dargestellt wird. Zwischen den beiden Klemmschenkeln 22 und 24 ist ein Klemmschenkelöffnungswinkelbegrenzer 40 aufgezeigt, der den relativen Öffnungswinkel begrenzen kann. An dieser Stelle sei nochmals darauf hingewiesen, dass der Begriff "unten" bei der Darstellung in Figur 2 auf die zum unteren Blattende führende Richtung bezieht, also auf die Richtung, in der sich die schwenkbaren Enden der Klemme 20 befinden.

Fig. 3 zeigt eine Draufsicht auf die Komponenten in einer ersten Schließposition (offene Klappenstellung). In dieser Ansicht ist die Schieberkulisse 1 in einem unbetätigten Zustand, bei im Wesentlichen geschlossener Klappe 14K dargestellt. Die Schieberkulisse 1 befindet sich nicht in Kontakt mit den Zapfen 14, aber drückt schon an die Schaltnocken 28 des Schließblechs 18. Der Griff 4 befindet sich in einer Komplett geöffneten Stellung, die ca. 85°-90° relativ einer Längsachse 8 der Schieberkulisse 1 erstreckt, wobei die Längsachse 8 längs der Klappe bzw. der Schieberkulisse 1 verläuft. Deutlich erkennbar ist in dieser Darstellung das gebogene Verriegelungselement (Anschlag) 16 an der Seite der Schieberkulisse 1 in einem Bereich, der der Infusionsschlauchklemme 20 an dem Gehäuse der Infusionspumpe 14g gegenüberliegt. Das Verriegelungselement 16 ist in der Form eines nach unten gebogenen Winkels ausgebildet, steht also wie ein Zahn von der riegelförmigen Schieberkulisse 1 vor. An der dem Schließblech 18 zugewandten Seite des Verriegelungselements 16 ist eine Schieberkulissenrampe 40 ausgebildet. Diese Schieberkulissenrampe 40 erstreckt sich in Längsrichtung der Längsachse in Richtung Griff 4 und ist an einem Längsende nach außen, in Richtung Klappenvorderseite geschwungen/abgeschrägt/gebogen.

Fig. 4 zeigt eine Schnittansicht durch die Vorrichtung in einer zweiten Schließposition (halboffene Klappenstellung). In dieser Position hat die Krafteinleitung durch den Klappengriff 4 begonnen. Das bedeutet, dass der Klappengriff 4 einen Winkel relativ zu der Schieberkulisse 1 einnimmt, der kleiner ist als seine Komplett offene Stellung ist, vorzugsweise kleiner als 85°, relativ zu der Längsachse der Schieberkulisse 1. Die Schieberkulisse 1 bewegt sich entlang der Längsachse 8 und kommt mit den Zapfen 12 in Eingriff. Die Zapfen 12 befinden sich somit im Fangbereich der Schieberkulisse 1, insbesondere der daran ausgebildeten Längsschlitze 10. Durch das in Eingriff kommen der Schieberkulisse 1 mit den Zapfen 12 bewegt sich die Schieberkulisse 1 und somit die Klappe 14k in Richtung des Infusionspumpengehäuses 14g, also in Schließrichtung der Klappe 14k. Durch die Bewegung der Klappe 14k in Richtung zum Infusionspumpengehäuse 14g drückt die Schieberkulisse 1 die Schaltnocke 28 und somit das Schließblech 18 mit dem ersten Klemmschenkel 22 gegen den zweiten Klemmschenkel 24. Die beiden Schenkel 22 und 24 schwenken/klappen dabei zusammen und beginnen den Infusionsschlauch 36 zu quetschen.

Der in der Fig. 4 dargestellte Öffnungswinkel der beiden Klemmschenkel beträgt in dieser Position ca. 6°. Der Schnappnase 26 ist noch nicht geschlossen/eingerastet.

Fig. 5 zeigt eine Draufsicht auf die Komponenten in einer zweiten Schließposition (halboffene Klappenstellung). In dieser Ansicht ist deutlich zu sehen, dass der Griff 4 sich nicht mehr in seiner komplett geöffneten Position befindet und die Zapfen 12 in dem Fangbereich der Führungsnuten/Schlitze 10 sind. Des Weiteren hat sich die Schieberkulisse 1 in Pfeilrichtung auf der Längsachse der Schieberkulisse 1 weiterbewegt im Vergleich zu Figur 3.

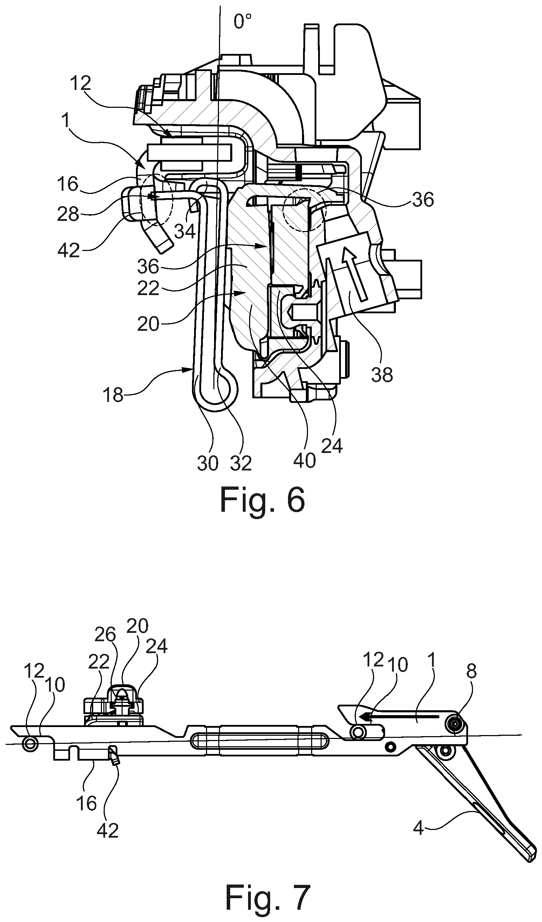

Fig. 6 zeigt eine Schnittansicht durch die Vorrichtung in einer dritten Schließposition (geschlossene Klappenstellung). Die Infusionsschlauchklemme 20 ist in dieser Position vollständig geschlossen. Das bedeutet, die beiden Klemmschenkel 22 und 24 haben den Infusionsschlauch 36 vollständig eingequetscht, so dass dieser kein Infusionsfluid durchlässt. Das Schließblech 18 ist durch das Verriegelungselement 16 druckbeaufschlagt. Die Schnappnase 26 ist in einer eingerasteten Position. In dieser Darstellung ist die Seitenansicht der Schieberkulissenrampe 42 zu sehen, an der die Schaltnocke 28 beginnt, an der Rampe 42 abzugleiten. Die Klappe 14k und somit die Schieberkulisse 1 sind nun in einer vollständig geschlossen Position relativ zu dem Infusionspumpengehäuse 14g.

Fig. 7 zeigt eine Draufsicht auf die Komponenten in einer dritten Schließposition (geschlossene Klappenstellung). Die Zapfen 12 gleiten in einen Langlochbereich der jeweiligen Führungsnuten/Schlitze 10, wobei der jeweilige Langlochbereich parallel zu der Längsachse 8 der Schieberkulisse 1 ausgerichtet ist. In dieser Position beginnt die Schaltnocke 28 - bedingt durch deren Geometrie - an der Schieberkulissenrampe 42 abzugleiten. Die Geometrie der Schaltnocke 28 umfasst eine Fläche, die parallel zur Längsachse 8 ausgerichtet ist und zwar in einem vorderen Bereich, der sich in Richtung zum Griff 4 befindet und weist danach eine Abschrägung an seinem hinteren Bereich auf.

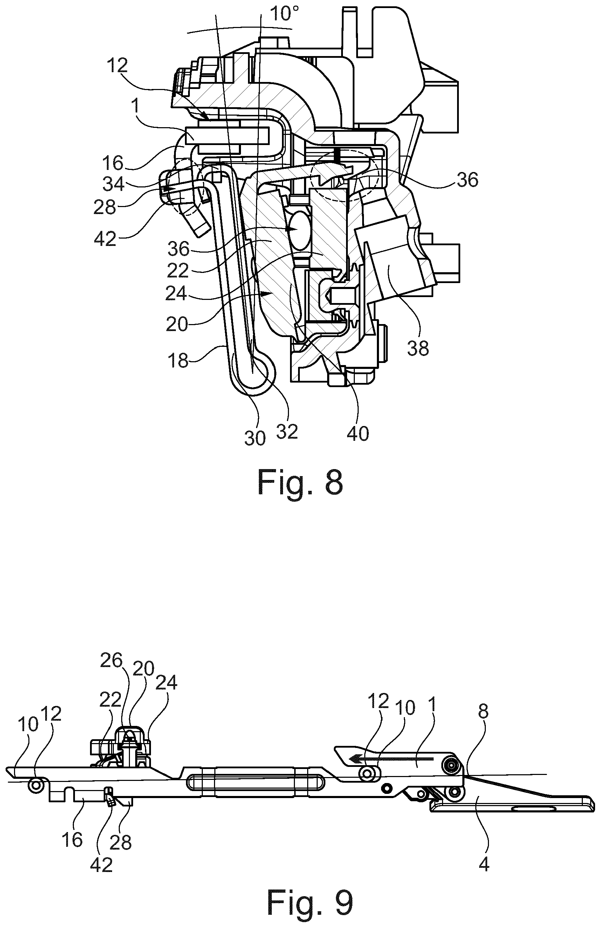

Fig. 8 zeigt eine Schnittansicht durch die Vorrichtung in einer vierten Schließposition (Klappenendstellung), also wenn sich der Griff 4, die Klappe 14k und die Schieberkulisse 1 in einer Endschließposition befinden. Die Schieberkulisse 1 ist dabei soweit in Pfeilrichtung/Schließrichtung der Längsachse 8 verfahren/verschoben, dass die Schaltnocke 28 an der Schieberkulissenrampe 42 soweit abgeglitten ist, dass sich beim Öffnen der Schnappnase 26 durch den Aktuator 38 das Schließblech 18 nicht mehr an der Rampe abstützt und somit der erste Klemmschenkel 22 aufklappen kann. Das Schließblech 18 wird sozusagen spannungsarm/spannungslos/spannungsfrei und drückt die beiden Klemmschenkel 22 und 24 nicht mehr zusammen. Durch das Öffnen der Infusionsschlauchklemme 20 wird der Infusionsschlauch nicht mehr oder wenig gequetscht/eingeklemmt und Infusionsfluid kann somit wieder durch diesen fließen.

Fig. 9 zeigt eine Draufsicht auf die Komponenten in der vierten Schließposition (Endklappenstellung). Ersichtlich ist hier besonders, dass sich der Griff 4 in der Endschließposition parallel zur Schieberkulisse befindet, die Schaltnocke 28 an der Schieberkulissenrampe 42 abgeglitten ist und das Schließblech 18 somit nicht mit Druck gegen den einen Klemmschenkel anliegt.

Zum Öffnen der Klappe 14k wird der Klappengriff 4 betätigt. Die Schieberkulisse 1 wird dadurch entgegen der Pfeilrichtung in Figur 9 gezogen. Dadurch gleitet die Schaltnocke 28 an der Schieberkulissenrampe 42 entlang. Die Infusionsschlauchklemme 20 wird somit beim Öffnen (Öffnungsvorgang) der Klappe 14k zwangsweise wieder verschlossen und die Klappe 14k kann komplett geöffnet werden. Somit wird automatisch beim Öffnen und Schließen der Klappe 14 die Infusionsschlauchklemme 20 immer verschlossen.

### Bezugszeichenliste

- 1: Schieberkulisse
- 2: Hebelgelenk
- 4: Klappengriff
- 6: Grifffeder
- 8: Längsachse
- 10: Führungsnuten
- 12: Zapfen
- 14k: Klappe
- 14p: Pumpengehäuse
- 16: Verriegelungselement
- 18: Schließblech
- 20: Infusionsschlauchklemme
- 22: Erster Klemmschenkel
- 24: Zweiter Klemmschenkel
- 26: Schnappnase/Schnapper
- 28: Schaltnocken
- 30: erste Schließblechschenkel
- 32: zweiter Schließblechschenkel
- 34: Umgriff
- 36: Infusionsschlauch
- 38: Aktuator
- 40: Klemmschenkelöffnungswinkelbegrenzer
- 42: Schieberkulissenrampe

## Patentansprüche

1. Schließmechanismus einer Schlauchklemme (20), aufweisend eine Schieberkulisse (1), die dafür ausgebildet ist, axial an einer schwenkbar an einem Gehäuse gehaltenen Klappe verschiebbar angeordnet oder gelagert zu sein, wobei die Schieberkulisse (1) bei einem axialen Bewegen der Schieberkulisse (1) in Richtung einer Schließposition zumindest einen am Gehäuse angeordneten/ausgebildeten Zapfen (12) oder Bolzen hinterschneidet und so mit dem axialen Bewegen in Richtung der Schließposition die Klappe gegen das Gehäuse zunehmend anzieht, um durch das axiale Bewegen der Schieberkulisse (1) relativ zur Klappe diese gegen das Gehäuse zu ziehen und zu verriegeln, wobei die im Gehäuse fixierte Schlauchklemme (20) einen ersten Klemmschenkel (22) und zweiten Klemmschenkel (24) aufweist, die an einem Ende schwenkbar miteinander verbunden sind, wobei die Schieberkulisse (1) wenigstens ein Verriegelungselement (16) hat, das zum Schließen der Schlauchklemme (20) während des axialen Bewegens der Schieberkulisse (1) vorgesehen ist,
**dadurch gekennzeichnet, dass**
ein **Betätigungselement** (18), das an dem ersten Klemmschenkel (22) angeordnet ist, zwischen der Schieberkulisse (1) und der Schlauchklemme (20) zur Übertragung einer Klemmkraft vom Verriegelungselement (16) auf die Schlauchklemme (20) angeordnet ist und eine **Federelastizität** mit bestimmter Steifigkeit aufweist, die so gewählt ist, dass bei Betätigen des Schließmechanismus eine Klemmkraft auf die Schlauchklemme (20) aufgebracht wird, die maximal zu einem vollständigen Abdrücken eines darin eingelegten Schlauchs (36) führt.

2. Schließmechanismus nach Anspruch 1, **dadurch gekennzeichnet, dass** der Schließmechanismus dafür vorbereitet und ausgebildet ist, dass bei bereits geschlossener Klappe die Schieberkulisse (1) eine spannungs- oder druckbeaufschlagende Stellung, bei welcher das vorzugsweise als Schließblech ausgebildete Betätigungselement (18) durch das Verriegelungselement oder den Verriegelungsabschnitt (16) der Schieberkulisse (1) in druckbeaufschlagenden Eingriff mit der Schlauchklemme (20) ist und eine spannungs- oder druckfreie Stellung annimmt, welche die, die Klappe verriegelnde Stellung darstellt und bei welcher das Betätigungselement (18) in drucklosem Kontakt mit der Schlauchklemme (20) ist, um ein Öffnen der Schlauchklemme (20) bei geschlossener Klappe zu ermöglichen.

3. Schließmechanismus nach Anspruch 2, **dadurch gekennzeichnet, dass** eine Schieberkulissenrampe (42) an dem Verriegelungselement (16) der Schieberkulisse (1) ausgebildet ist, an der ein Schaltnocken (28) des Betätigungselements (18) gleitend anliegt, derart, dass bei einer Längsverschiebung der Schieberkulisse (1) aus der spannungs- oder druckbeaufschlagende Stellung in Richtung spannungs- oder druckfreie Stellung die Klemmkraft kontinuierlich zunimmt und mit Erreichen der spannungs- oder druckfreien Stellung von der Schieberkulissenrampe (42) abgleitet.

4. Schließmechanismus nach einem der vorgehenden Ansprüche, **dadurch gekennzeichnet, dass** das Verriegelungselement oder der Verriegelungsabschnitt (16) an der Schieberkulisse (1) in Form eines Winkelelements ausgebildet ist, vorzugsweise mit einem Winkel von im Wesentlichen 90° zur Längsachse (8) der Schieberkulisse (1).

5. Schließmechanismus nach einem der vorgehenden Ansprüche, **dadurch gekennzeichnet, dass** das Betätigungselement (18) ein, vorzugsweise einstückiges Federblech ist, das im Querschnitt eine V- oder U-Form hat.

6. Infusionspumpe mit einem Pumpengehäuse, an dem eine Klappe anscharniert ist, und welche in geöffnetem Zustand einen Zugang in das Innere des Pumpengehäuses frei gibt, um das Einsetzen einer Schlauchklemme (20) vorzugsweise der Einwegbauart in das Pumpengehäuse und das Einlegen eines Schlauchs (36) in die eingesetzte Schlauchklemme (20) zu ermöglichen, **gekennzeichnet durch** einen Schließmechanismus mit den Merkmalen gemäß einem der Ansprüche 1 bis 5.

7. Verfahren zum Öffnen und Schließen einer in eine Pumpe vorzugsweise gemäß Anspruch 6 eingesetzten Schlauchklemme (20) unter Verwendung eines Schließmechanismus vorzugsweise gemäß einem der Ansprüche 1 bis 5, wonach die Schlauchklemme (20) beim Verschließen und/oder beim Öffnen der Klappe geschlossen wird und in einer Endschließposition des Schließmechanismus geöffnet werden kann,
**dadurch gekennzeichnet, dass**
die Klappe geschlossen wird und ein Verriegelungselement (16) einer axial verschiebbar an der Klappe gehaltenen Schieberkulisse (1) gegen einen Schaltnocken (28) eines Schließblechs (18) drückt, wodurch die Schlauchklemme (20) geschlossen wird, wofür
der Schaltnocken (28) mit oder kurz vor Erreichen der Endschließposition des Schließmechanismus, insbesondere der Schieberkulisse (1) an einer daran angeordneten oder ausgebildeten Schieberkulissenrampe (42) abgleitet, wodurch die Kraftbeaufschlagung auf das Schließblech (18) abbricht, und schließlich
ein Aktuator (38) die Rastnase (26) an dem oberen Ende der Schlauchklemme (20) in der Endschließposition des Schließmechanismus, insbesondere der Schieberkulisse (1), in der der erste Klemmschenkel (22) mit dem zweiten Klemmschenkel (24) verrastet ist, öffnen kann, wobei
die Schlauchklemme (20) beim Öffnen der Klappe durch die reverse axiale Verschiebung der Schieberkulisse (1) durch Aufgleiten der Schaltnocke (28) auf die Schieberkulissenrampe (42) zwangsweise geschlossen wird.

## Claims

1. A closing mechanism of a tube clamp (20) comprising a slide link (1) which is prepared and configured to be movably arranged or supported axially on a flap pivoted on a casing, the slide link (1) undercutting at least one pin (12) or bolt arranged/formed on the casing when the slide link (1) is moved axially in the direction of a locking position and thus increasingly pulling the flap against the casing with the axial movement in the direction of the locking position in order to pull and lock the slide link (1) against the casing by moving it axially relative to the flap, a tube clamp (20) fixed in the casing having a first clamping leg (22) and a second clamping leg (24) which are pivotably connected to one another at one end, the slide link (1) including at least one locking element (16) provided for closing the tube clamp (20) while the slide link (1) is axially moved,
**characterized in that**
an **actuating element** (18), which is arranged on the first clamping leg (22), is arranged between the slide link (1) and the tube clamp (20) for transmitting a clamping force from the locking element (16) to the tube clamp (20) and has a **spring elasticity** with a specific stiffness which is selected such that when the closing mechanism is actuated, a clamping force is applied to the tube clamp (20), which at the maximum leads to complete squeezing of a tube (36) inserted therein.

2. The closing mechanism according to claim 1, **characterized in that** the closing mechanism is prepared and configured so that, when the flap is already closed, the slide link (1) adopts a stress-applying or pressurizing position at which the actuating element (18) preferably in the form of a keeper plate is in pressurizing engagement with the tube clamp (20) by the locking element or the locking portion (16) of the slide link (1) and adopts a stress-free or pressure-free position which constitutes the position locking the flap and at which the actuating element (18) is in pressure-less contact with the tube clamp (20) so as to enable opening of the tube clamp (20) while the flap is closed.

3. The closing mechanism according to claim 2, **characterized in that** a slide link ramp (42) is formed on the locking element (16) of the slide link (1), to which a switching cam (28) of the actuating element (18) is slidingly adjacent or abuts such that when the slide link (1) is longitudinally displaced from the tensioning or pressurizing position in the direction of the stress-free or pressure-free position, the clamping force continuously increases and, upon reaching the tension-free or pressure-free position, slides or slips off the slide link ramp (42).

4. The closing mechanism according to any of the preceding claims, **characterized in that** the locking element or the locking portion (16) is configured a the slide link (1) in the form of an angular element, preferably having an angle of substantially 90° relative to the longitudinal axis (8) of the slide link (1).

5. The closing mechanism according to any one of the preceding claims,
**characterized in that** the actuating element (18) preferably is a one-part spring plate, being V or U-shaped in cross-section.

6. An infusion pump comprising a pump casing to which a flap is hinged and which in the opened state releases access to the interior of the pump casing so as to enable insertion of a tube clamp (20) preferably of the single-use type into the pump casing and to enable insertion of a tube (36) into the inserted tube clamp (20),
**characterized by** a closing mechanism comprising the features according to any one of claims 1 to 5.

7. A method of opening and closing a tube clamp (20) inserted in a pump preferably according to claim 6, using a closing mechanism preferably according to any one of claims 1 to 5, according to which the tube clamp (20) is closed upon closing and/or opening the flap and can be opened in a final closing position of the closing mechanism,
**characterized in that**
the flap is closed and a locking element (16) of a slide link (1) held to be axially movable at the flap presses against a switching cam (28) of a keeper plate (18), thus causing the tube clamp (20) to be closed, for which purpose the switching cam (28), upon or shortly before reaching the final closing position of the closing mechanism, especially of the slide link (1), slides off a slide link ramp (42) arranged or configured on the slide link, thus causing the application of force to the keeper plate (18) to stop, and finally
an actuator (38) can open the snap lug (26) at the upper end of the tube clamp (2) in the final closing position of the closing mechanism, especially of the slide link (1), in which the first clamping leg (22) snaps into place with the second clamping leg (24) wherein
the tube clamp (20), upon opening of the flap by the reverse axial displacement of the slide link (1), is closed by force by the switching cam (28) sliding onto the slide link ramp (42).

## Revendications

1. Mécanisme de fermeture d'une pince de tuyau (20) présentant un coulisseau (1) qui est réalisé afin d'être agencé ou logé de manière mobile axialement au niveau d'un clapet maintenu de manière pivotante au niveau d'un boîtier, dans lequel le coulisseau (1) contre-dépouille lors d'un déplacement axial du coulisseau (1) en direction d'une position de fermeture au moins un tenon (12) ou boulon agencé/réalisé au niveau du boîtier et attire ainsi de plus en plus le clapet contre le boîtier avec le déplacement axial en direction de la position de fermeture afin de tirer par le déplacement axial du coulisseau (1) par rapport au clapet celui-ci contre le boîtier et de le verrouiller, dans lequel la pince de tuyau (20) fixée dans le boîtier présente une première branche de serrage (22) et une seconde branche de serrage (24) qui sont reliées entre elles de manière pivotante à une extrémité, dans lequel le coulisseau (1) présente au moins un élément de verrouillage (16) qui est prévu pour la fermeture de la pince de tuyau (20) pendant le déplacement axial du coulisseau (1),
**caractérisé en ce que**
**unélément d'actionnement** (18) qui est agencé au niveau de la première branche de serrage (22), est agencé entre le coulisseau (1) et la pince de tuyau (20) pour la transmission d'une force de serrage de l'élément de verrouillage (16) à la pince de tuyau (20) et présente une **élasticité de ressort** avec une rigidité déterminée qui est choisie de sorte que, lors de l'actionnement du mécanisme de fermeture, une force de serrage soit appliquée à la pince de tuyau (20) qui mène au maximum à un cisaillement complet d'un tuyau (36) introduit à l'intérieur.

2. Mécanisme de fermeture selon la revendication 1, **caractérisé en ce que** le mécanisme de fermeture est préparé et réalisé afin que, dans le cas où le clapet est déjà fermé, le coulisseau (1) adopte une position d'application de tension ou de pression, pour laquelle l'élément d'actionnement (18) réalisé de préférence comme tôle de fermeture est en prise d'application de pression avec la pince de tuyau (20) par l'élément de verrouillage ou la section de verrouillage (16) du coulisseau (1), et une position sans tension ou sans pression qui représente la position verrouillant le clapet et pour laquelle l'élément d'actionnement (18) est en contact sans pression avec la pince de tuyau (20) afin de permettre une ouverture de la pince de tuyau (20) dans le cas où le clapet est fermé.

3. Mécanisme de fermeture selon la revendication 2, **caractérisé en ce qu'**une rampe de coulisseau (42) est réalisée au niveau de l'élément de verrouillage (16) du coulisseau (1), au niveau duquel une came de commutation (28) de l'élément d'actionnement (18) repose par glissement de telle manière que, lors d'un déplacement longitudinal du coulisseau (1) à partir de la position d'application de tension ou de pression en direction de la position sans tension ou sans pression, la force de serrage augmente en continu et glisse lors de l'atteinte de la position sans tension ou sans pression de la rampe de coulisseau (42).

4. Mécanisme de fermeture selon l'une quelconque des revendications précédentes, **caractérisé en ce que** l'élément de verrouillage ou la section de verrouillage (16) est réalisé au niveau du coulisseau (1) sous la forme d'un élément angulaire, de préférence avec un angle de sensiblement 90° par rapport à l'axe longitudinal (8) du coulisseau (1).

5. Mécanisme de fermeture selon l'une quelconque des revendications précédentes, **caractérisé en ce que** l'élément d'actionnement (18) est une tôle à ressort de préférence d'un seul tenant qui présente en section transversale une forme en V ou U.

6. Pompe d'infusion avec un boîtier de pompe, auquel un clapet est articulé, et qui libère dans l'état ouvert un accès à l'intérieur du boîtier de pompe afin de permettre l'insertion d'une pince de tuyau (20) de préférence du type à usage unique dans le boîtier de pompe et l'introduction d'un tuyau (36) dans la pince de tuyau (20) insérée, **caractérisée par** un mécanisme de fermeture avec les caractéristiques selon l'une quelconque des revendications 1 à 5.

7. Procédé d'ouverture et de fermeture d'une pince de tuyau (20) insérée dans une pompe de préférence selon la revendication 6 en utilisant un mécanisme de fermeture de préférence selon l'une quelconque des revendications 1 à 5, après quoi la pince de tuyau (20) est fermée lors de la fermeture et/ou lors de l'ouverture du clapet et peut être ouverte dans une position de fermeture finale du mécanisme de fermeture,
**caractérisé en ce que**
le clapet est fermé et un élément de verrouillage (16) d'un coulisseau (1) maintenu de manière axialement mobile sur le clapet presse contre une came de commutation (28) d'une tôle de fermeture (18), selon lequel la pince de tuyau (20) est fermée, ce pour quoi
la came de commutation (28) glisse lors de ou juste avant l'atteinte de la position de fermeture finale du mécanisme de fermeture, en particulier du coulisseau (1) sur une rampe de coulisseau (42) réalisée ou agencée dessus, selon lequel l'application d'une force s'interrompt sur la tôle de fermeture (18), et enfin
un actionneur (38) peut ouvrir le nez d'encliquetage (26) au niveau de l'extrémité supérieure de la pince de tuyau (20) dans la position de fermeture finale du mécanisme de fermeture, en particulier du coulisseau (1), dans lequel la première branche de serrage (22) est encliquetée avec la seconde branche de serrage (24), dans lequel
la pince de tuyau (20) est fermée de manière forcée lors de l'ouverture du clapet par le déplacement axial inverse du coulisseau (1) par glissement de la came de commutation (28) sur la rampe de coulisseau (42).
